# EUROPÄISCHE PATENTSCHRIFT

(11) **EP 0 414 019 B1**
(45) Veröffentlichungstag und Bekanntmachung des Hinweises auf die Patenterteilung: **29.05.1996**
(21) Anmeldenummer: 90115022.7
(22) Anmeldetag: 04.08.1990
(51) Int. Cl.: C07D 215/14, A61K 31/47

(54) **Substituierte N-(Chinolin-2-yl-methoxy)benzylsulfonylharnstoffe**
Subsituted N-(quinolin-2-yl-methoxy)benzylsulfonylureas
N-(Quinoléine-2-yl-méthoxy)benzylsulfonylurées substituées

(30) Priorität: 19.08.1989 DE 3927369
(43) Veröffentlichungstag der Anmeldung: 27.02.1991
(73) Patentinhaber: BAYER AG, 51368 Leverkusen (DE)
(72) Erfinder: Mohrs, Klaus, Dr., D-5600 Wuppertal 1 (DE); Raddatz, Siegfried, Dr., D-5000 Köln 80 (DE); Fruchtmann, Romanis, D-5000 Köln 1 (DE); Kohlsdorfer, Christian, Dr., D-5042 Erfstadt (DE); Müller-Peddinghaus, Reiner, Prof. Dr., D-5060 Bergisch-Gladbach 2 (DE); Theisen-Popp, Pia, Dr., D-5060 Bergisch-Gladbach 2 (DE)

(56) Entgegenhaltungen:
- EP-A- 0 029 982
- EP-A- 0 232 954
- EP-A- 0 261 539
- WO-A-88/06886
- US-A- 4 826 987

## Beschreibung

Die Erfindung betrifft substituierte N-(Chinolin-2-yl-methoxy)benzyl-sulfonylharnstoffe, Verfahren zu ihrer Herstellung und ihre Verwendung in Arzneimitteln.

Es ist bekannt, daß Sulfonylharnstoffe eine antidiabetische Wirkung besitzen (vgl. W. Forth, Allgemeine und Spezielle Pharmakologie und Toxikologie, 4. Aufl., 1983, B.I.-Wissenschaftsverlag). Ferner sind N,N-Dimethyl-N′-[3-(2-chinolyl-methoxy)phenyl-harnstoffe in JP 82 64 675, Appl. 80/140 091 beschrieben worden.

Außerdem werden im US-Patent 4 826 987 Pyridyl- und Chinolylamine beschrieben, die eine spezifische 5-Lipoxygenase-Inhibition aufweisen.

Es wurden nun N-(Chinolin-2-yl-methoxy)benzyl-sulfonylharnstoffe der allgemeinen Formel (I), 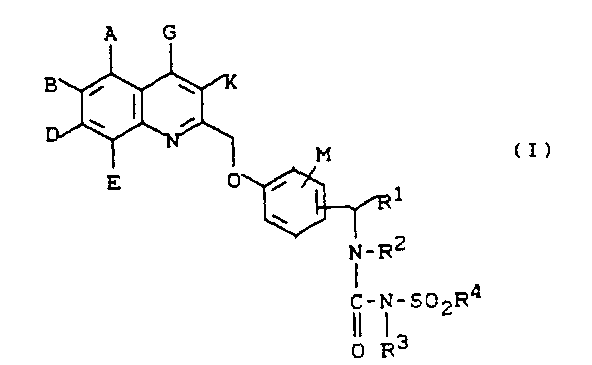 in welcher
A, B, D, E, G, K und M gleich oder verschieden sind und
- für Wasserstoff, Hydroxy, Halogen, Trifluormethyl, Trifluormethoxy oder Carboxy stehen,
- für geradkettiges oder verzweigtes Alkyl mit bis zu 10 Kohlenstoffatomen stehen, das gegebenenfalls durch Hydroxy oder Halogen substituiert ist,
- für geradkettiges oder verzweigtes Alkoxy oder Alkoxycarbonyl mit bis zu 10 Kohlenstoffatomen stehen, oder
- für Aryl mit 6 bis 10 Kohlenstoffatomen stehen, das gegebenenfalls durch Halogen, Nitro, Cyano oder durch geradkettiges oder verzweigtes Alkyl oder Alkoxy mit bis zu 8 Kohlenstoffatomen substituiert ist,

- R¹: - für geradkettiges oder verzweigtes Alkyl mit bis zu 10 Kohlenstoffatomen steht, das gegebenenfalls durch Alkoxy mit bis zu 8 Kohlenstoffatomen, Halogen oder durch Cycloalkyl mit 3 bis 8 Kohlenstoffatomen substituiert ist,
- für Cycloalkyl mit 3 bis 8 Kohlenstoffatomen steht, das gegebenenfalls durch Halogen oder geradkettiges oder verzweigtes Alkyl mit bis zu 8 Kohlenstoffatomen substituiert ist,
- R und R³: gleich oder verschieden sind und
- für Wasserstoff oder geradkettiges oder verzweigtes Alkyl mit bis zu 10 Kohlenstoffatomen stehen, das gegebenenfalls durch Halogen, Hydroxy oder durch Aryl mit 6 bis 10 Kohlenstoffatomen substituiert ist,
- R⁴: - für geradkettiges oder verzweigtes Alkyl mit bis zu 10 Kohlenstoffatomen steht, das gegebenenfalls durch Halogen, Nitro, Cyano, Hydroxy, Trifluormethyl oder durch Aryl mit 6 bis 10 Kohlenstoffatomen substituiert ist,
- für einen 5- bis 7-gliedrigen Heterocyclus mit bis zu 4 verschiedenen Heteroatomen aus der Reihe Schwefel, Sauerstoff oder Stickstoff, oder für Aryl mit 6 bis 10 Kohlenstoffatomen steht, wobei der Heterocyclus und der Arylrest gegebenenfalls bis zu 3-fach gleich oder verschieden durch Halogen, Nitro, Cyano, Hydroxy, geradkettiges oder verzweigtes Alkyl, Alkylthio oder Alkoxy mit bis zu 8 Kohlenstoffatomen, Trifluormethyl oder Trifluormethoxy substituiert sind,
und deren Salze gefunden.

Im Rahmen der vorliegenden Erfindung werden physiologisch unbedenkliche Salze bevorzugt. Physiologisch unbedenkliche Salze der substituierten N-(Chinolin-2-yl-methoxy)benzyl-sulfonylharnstoffe können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Salze im Rahmen der vorliegenden Erfindung sind außerdem Salze der einwertigen Metalle wie Alkalimetalle und die Ammoniumsalze. Bevorzugt werden Natrium-, Kalium- und Ammoniumsalze.

Bevorzugt sind Verbindungen der allgemeinen Formel (I),
in welcher
A, B, D, E, G, K und M gleich oder verschieden sind und
- für Wasserstoff, Fluor, Chlor, Trifluormethoxy oder Carboxy stehen,
- für geradkettiges oder verzweigtes Alkyl mit bis zu 8 Kohlenstoffatomen stehen, das gegebenenfalls durch Hydroxy, Fluor, Chlor oder Brom substituiert ist,
- für geradkettiges oder verzweigtes Alkoxy oder Alkoxycarbonyl mit bis zu 8 Kohlenstoffatomen stehen, oder
- für Phenyl stehen, das gegebenenfalls durch Fluor, Chlor, Brom, Nitro, Cyano oder durch geradkettiges oder verzweigtes Alkyl oder Alkoxy mit bis zu 6 Kohlenstoffatomen substituiert ist,

- R¹: - für geradkettiges oder verzweigtes Alkyl mit bis zu 8 Kohlenstoffatomen steht, das gegebenenfalls durch Hydroxy, Fluor, Chlor, Alkoxy mit bis zu 6 Kohlenstoffatomen oder durch Cyclopropyl, Cyclobutyl, Cyclopentyl, Cyclohexyl oder Cycloheptyl substituiert ist,
- für Cyclopropyl, Cyclobutyl, Cyclopentyl, Cyclohexyl oder Cycloheptyl steht, das gegebenenfalls durch Fluor, Chlor oder geradkettiges oder verzweigtes Alkyl mit bis zu 6 Kohlenstoffatomen substituiert ist,
- R und R³: gleich oder verschieden sind und
- für Wasserstoff oder geradkettiges oder verzweigtes Alkyl mit bis zu 8 Kohlenstoffatomen stehen, das gegebenenfalls durch Fluor oder Chlor substituiert ist,
- R⁴: - für geradkettiges oder verzweigtes Alkyl mit bis zu 8 Kohlenstoffatomen steht, das gegebenenfalls durch Fluor, Chlor, Nitro, Cyano, Hydroxy oder durch Phenyl substituiert ist,
- für Phenyl steht, das gegebenenfalls bis zu 2-fach gleich oder verschieden durch Fluor, Chlor, Brom, Nitro, Cyano, Hydroxy, geradkettiges oder verzweigtes Alkyl, Alkylthio oder Alkoxy mit bis zu 6 Kohlenstoffatomen, Trifluormethyl oder Trifluormethoxy substituiert ist
und deren Salze.

Besonders bevorzugt sind Verbindungen der allgemeinen Formel (I)
in welcher
A, B, D, E, G, K und M gleich oder verschieden sind und
- für Wasserstoff, Fluor, Chlor, geradkettiges oder verzweigtes Alkyl oder Alkoxy mit bis zu 6 Kohlenstoffatomen stehen,

- R¹: - für geradkettiges oder verzweigtes Alkyl mit bis zu 6 Kohlenstoffatomen steht, das gegebenenfalls durch Fluor, Chlor oder Alkoxy mit bis zu 4 Kohlenstoffatomen oder durch Cyclopropyl, Cyclopentyl oder Cyclohexyl substituiert ist,
- für Cyclobutyl, Cyclopentyl, Cyclohexyl oder Cycloheptyl steht, das gegebenenfalls durch geradkettiges oder verzweigtes Alkyl mit bis zu 4 Kohlenstoffatomen oder durch Fluor substituiert ist,
- R und R³: gleich oder verschieden sind und
- für Wasserstoff oder geradkettiges oder verzweigtes Alkyl mit bis zu 6 Kohlenstoffatomen stehen,
- R⁴: - für geradkettiges oder verzweigtes Alkyl mit bis zu 6 Kohlenstoffatomen steht, das gegebenenfalls durch Fluor, Chlor oder Phenyl substituiert ist,
- für Phenyl steht, das gegebenenfalls durch Fluor, Chlor, Brom oder durch geradkettiges oder verzweigtes Alkyl mit bis zu 4 Kohlenstoffatomen substituiert ist
und deren Salze.

Ganz besonders bevorzugt sind Verbindungen der allgemeinen Formel (I), in denen die Chinolylmethoxygruppierung am Phenylring in 4-Stellung zur N-substituierten Sulfonylharnstoffgruppe steht.

Außerdem wurde ein Verfahren zur Herstellung der erfindungsgemäßen Verbindungen der allgemeinen Formel (I) 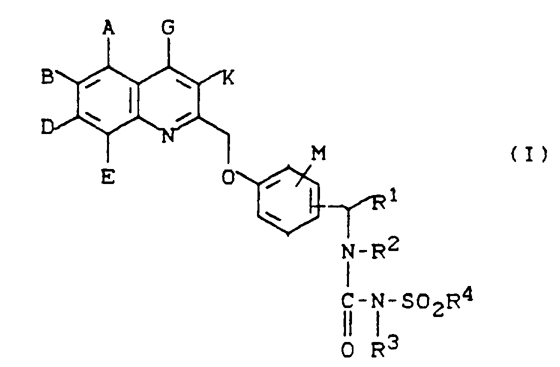 in welcher
A, B, D, E, G, K, M, R¹, R, R³ und R⁴ die oben angegebene Bedeutung haben,
gefunden, das dadurch gekennzeichnet ist, daß man
Amine der allgemeinen Formel (II) 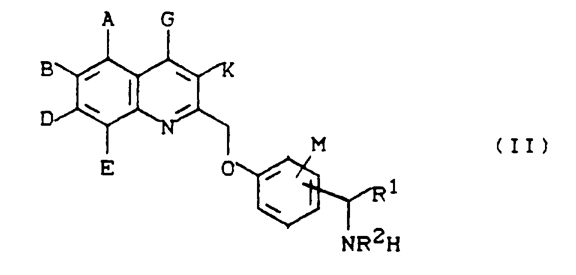 in welcher
A, B, D, E, G, K, M, R¹ und R die oben angegebene Bedeutung haben,
mit Sulfonylisocyanaten der allgemeinen Formel (III)

   R⁴-SO₂-NCO (III)

   in welcher
R⁴ die oben angegebene Bedeutung hat,
in einem inerten Lösemittel zu Verbindungen der allgemeinen Formel (Ia) 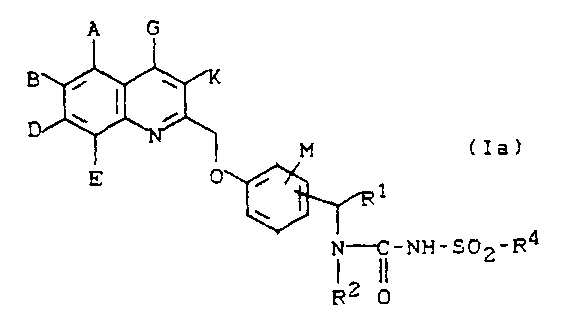 in welcher
A, B, D, E, G, K, M, R¹, R und R⁴ die oben angegebene Bedeutung haben,
umsetzt, und im Fall der Verbindungen der Formel (I) mit R³ ≠ H anschließend die Verbindungen der Formel (Ia) mit Alkylierungsmitteln in inerten Lösemitteln alkyliert.

Das erfindungsgemäße Verfahren kann durch folgendes Formelschema erläutert werden:

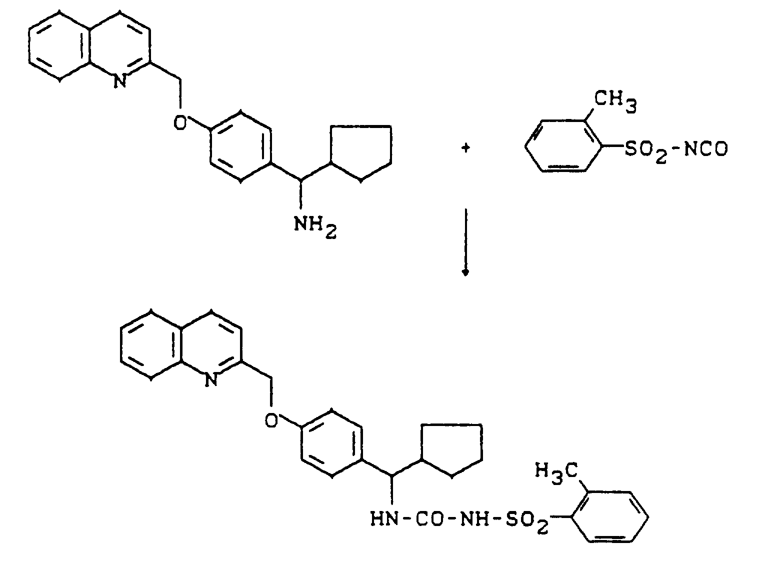

Als Lösemittel für das erfindungsgemäße Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dichlormethan.

Das erfindungsgemäße Verfahren wird im allgemeinen in einem Temperaturbereich von -80°C bis +80°C, bevorzugt von -80°C bis 0°C durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Im allgemeinen setzt man 1 bis 3 Mol, bevorzugt 1 bis 2 Mol, besonders bevorzugt 1 Mol Sulfonylisocyanat, bezogen auf 1 Mol des Amins ein.

Die Sulfonylisocyanate der allgemeinen Formel (III) sind bekannt oder können nach bekannten Methoden hergestellt werden [C. King, J. Org. Chem. 25, 352 (1960); F. Effenberger, R. Gleiter, Chem. Ber. 97, 1576 (1964); H. Ulrich, A.A.R. Sayigh, Angew. Chem. 78, 761 (1966); Houben-Weyl VIII, 128].

Als Alkylierungsmittel bei dem Verfahren können beispielsweise (C₁-C₈)-Alkylhalogenide, Sulfonsäureester oder substituierte oder unsubstituierte (C₁-C₆)-Dialkyl- oder (C₆-C₁₀)-Diarylsulfate, vorzugsweise Methyljodid, p-Toluolsulfonsäureester oder Dimethylsulfat eingesetzt werden.

Die Alkylierung wird in den oben aufgeführten Lösemitteln bei Temperaturen von 0°C bis +150°C, vorzugsweise bei Raumtemperaturen bis +100°C bei Normaldruck durchgeführt.

Die Amine der allgemeinen Formel (II) sind teilweise bekannt (R¹ = (CH₂)₄CH₃) und können außerdem hergestellt werden, indem man
Verbindungen der allgemeinen Formel (IV) 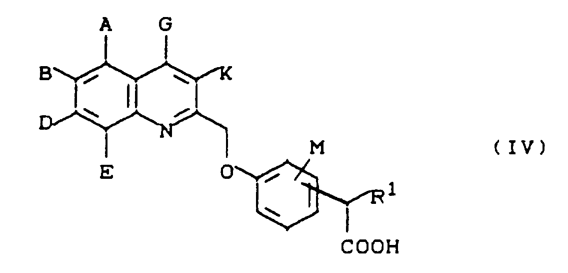 in welcher
A, B, D, E, G, K, M und R¹ die oben angegebene Bedeutung haben,
zunächst mit Aziden, wie beispielsweise Phosphorsäurdiphenylesterazid oder Stickstoffwasserstoffsäure, bevorzugt mit Phosphorsäurediphenylesterazid, in inerten Lösemitteln, gegebenenfalls in Anwesenheit einer Base und anschließend mit Säuren nach der üblichen Methode (Überführung einer Carbonsäure zum entsprechenden Amin) unter Abspaltung von Stickstoff und Kohlendioxid umsetzt,
und im Fall, daß in den Aminen der allgemeinen Formel (II) R ≠ H ist, die Aminogruppe nach der oben aufgeführten Methode alkyliert.

Das Verfahren kann durch folgendes Formelschema erläutert werden:

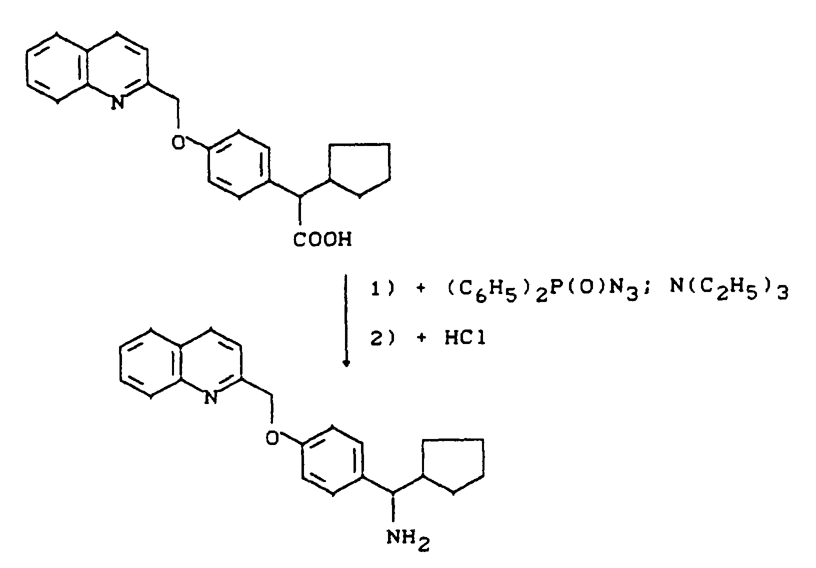

Als Lösemittel für den Säureabbau eignen sich die oben aufgeführten inerten Lösemittel. Bevorzugt ist Dimethylformamid.

Als Basen eignen sich organische Amine (Trialkyl(C₁-C₆)amine, wie beispielsweise Triethylamin oder Heterocyclen wie Pyridin, Methylpiperidin, Piperidin oder Morpholin. Bevorzugt ist Triethylamin.

Als Säuren werden im allgemeinen Mineralsäuren eingesetzt. Bevorzugt werden hierbei Chlorwasserstoffsäure, Bromwasserstoffsäure, Schwefelsäure, Phosphorsäure oder aber Gemische der genannten Säuren eingesetzt.

Die Reaktion erfolgt in einem Temperaturbereich von 0°C bis +130°C, bevorzugt von 0°C bis +80°C.

Die Umsetzung wird im allgemeinen bei Normaldruck durchgeführt. Es ist aber auch möglich, das Verfahren bei Unterdruck oder Überdruck durchzuführen (z.B. in einem Bereich von 0,5 bis 5 bar).

Phosphorsäurdiphenylesterazid ist bekannt [vgl. J. Am. Chem. Soc. 94, 6203 (1972); J. Org. Chem. 39, 2302 (1974); Tetrahedron Lett. 1977 (1977)].

Die Verbindungen der allgemeinen Formel (IV) sind neu und können beispielsweise hergestellt werden, indem man
Verbindungen der allgemeinen Formel (V) 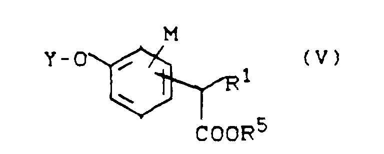 in welcher
- R¹ und M: die oben angegebene Bedeutung haben,
- R⁵: - für Wasserstoff, Phenyl oder C₁-C₆-Alkyl steht
und
- Y: - für eine typische Hydroxyschutzgruppe wie beispielsweise Benzyl oder tert.Butyl steht, nach Abspaltung der Schutzgruppe Y nach üblicher Methode mit Halogenmethylchinolinen der Formel (VI) 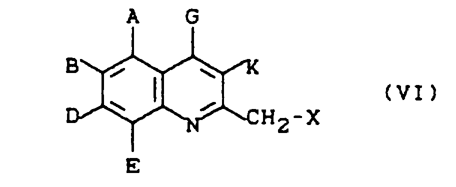
in welcher
A, B, D, G und K die oben angegebene Bedeutung haben
und
- X: - für Halogen steht,
verethert und im Fall der Säuren verseift.

Die Abspaltung der Schutzgruppen aus den entsprechenden Ethern erfolgt nach üblicher Methode, beispielsweise durch hydrogenolytische Spaltung der Benzylether in den oben aufgeführten inerten Lösemitteln in Anwesenheit eines Katalysators mit Wasserstoff-Gas [vgl. außerdem Th. Greene: "Protective Groups in Organic Synthesis", J. Wiley & Sons, 1981, New York].

Die Veretherung kann in inerten organischen Lösemitteln gegebenenfalls in Anwesenheit einer Base durchgeführt werden.

Lösemittel für die Veretherung können inerte organische Lösemittel sein, die sich unter den Reaktionsbedingungen nicht verändern. Hierzu gehören vorzugsweise Ether wie beispielsweise Dioxan, Tetrahydrofuran oder Diethylether, Halogenkohlenwasserstoffe wie Dichlormethan, Trichlormethan, Tetrachlormethan, 1,2-Dichlorethan oder Trichlorethylen, Kohlenwasserstoffe wie Benzol, Xylol, Toluol, Hexan, Cyclohexan oder Erdölfraktionen, Nitromethan, Dimethylformamid, Acetonitril, Aceton oder Hexamethylphosphorsäuretriamid. Ebenso ist es möglich, Gemische der Lösemittel einzusetzen.

Als Basen für die Veretherung können anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder organische Amine (Trialkyl(C₁-C₆)amine) wie Triethylamin, oder Heterocyclen wie Pyridin, Methylpiperidin, Piperidin oder Morpholin.

Es ist auch möglich, als Basen Alkalimetalle wie Natrium und deren Hydride, wie Natriumhydrid, einzusetzen.

Die Veretherung erfolgt im allgemeinen in einem Temperaturbereich von 0°C bis +150°C, bevorzugt von +10°C bis +100°C.

Die Veretherung wird im allgemeinen Normaldruck durchgeführt. Es ist aber auch möglich, das Verfahren bei Unterdruck oder Überdruck durchzuführen (z.B. in einem Bereich von 0,5 bis 5 bar).

Im allgemeinen setzt man 0,5 bis 5, bevorzugt 1 bis 2 Mol Halogenid, bezogen auf 1 Mol des Reaktionspartners ein. Die Base wird im allgemeinen in einer Menge von 0,5 bis 5 Mol, bevorzugt von 1 bis 3 Mol bezogen auf das Halogenid eingesetzt.

Die Verbindungen der allgemeinen Formel (V) sind an sich bekannt oder können nach üblicher Methode hergestellt werden [vgl. A. Ulrich, B. Tucker, A.A. R. Sayigh, J. Org. Chem. 31, 2658 (1966)].

Ebenfalls sind die Verbindungen der allgemeinen Formel (VI) und ihre Darstellung bekannt.

Beispielsweise können folgende Halogenide erfindungsgemäß verwendet werden:
8-Chlor-2-chlormethyl-chinolin
7-Chlor-2-chlormethyl-chinolin
6-Fluor-2-chlormethyl-chinolin

Die Verseifung der Carbonsäureester erfolgt nach üblichen Methoden, indem man die Ester in inerten Lösemitteln mit üblichen Basen behandelt, wobei die zunächst entstehenden Salze durch Behandeln mit Säure in die freien Carbonsäuren überführt werden können.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat, Kaliumethanolat, Kaliummethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters eingesetzt. Besonders bevorzugt verwendet man molare Mengen der Reaktanden.

Bei der Durchführung der Reaktion entstehen im ersten Schritt die Salze der erfindungsgemäßen Verbindungen als Zwischenprodukte, die isoliert werden können. Die erfindungsgemäßen Säuren erhält man durch Behandeln der Salze mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren hierbei als vorteilhaft erwiesen, die basische Reaktionsmischung der Verseifung in einem zweiten Schritt ohne Isolierung der Salze anzusäuern. Die Säuren können dann in üblicher Weise isoliert werden.

Die erfindungsgemäßen Verbindungen der allgemeinen Formel (I) zeigen überraschenderweise eine hohe in vitro Aktivität als Leukotriensynthesehemmer und eine starke in vivo Wirkung nach oraler Applikation.

Die erfindungsgemäßen substituierten N-(Chinolyl-2-yl-methoxy)benzyl-sulfonylharnstoffe können als Wirkstoffe in Arzneimitteln eingesetzt werden. Die Stoffe können als Hemmer von enzymatischen Reaktionen im Rahmen des Arachidonsäurestoffwechsels, insbesondere der 5-Lipoxygenase, wirken.

Sie sind somit bevorzugt zur Behandlung und Verhütung von Erkrankungen der Atemwege wie Allergien/Asthma, Bronchitis, Emphysema, Schocklunge, pulmonaler Hypertonie, Entzündungen/Rheuma und Ödemen, Thrombosen und Thromboembolien, Ischämien (periphere, cardiale, cerebrale Durchblutungsstörungen), Herz- und Hirninfarkten, Herzrhythmusstörungen, Angina pectoris, Arteriosklerose, bei Gewebstransplantationen, Dermatosen wie Psoriasis, entzündliche Dermatosen, z.B. Ekzem, Dermatophyteninfektion, Infektionen der Haut durch Bakterien, Metastasen und zur Cytoprotection im Gastrointestinaltrakt geeignet.

Die erfindungsgemäßen substituierten N-(Chinolyl-2-yl-methoxy)benzyl-sulfonylharnstoffe können sowohl in der Humanmedizin als auch in der Veterinärmedizin angewendet werden.

Die pharmakologischen Wirkdaten der erfindungsgemäßen Substanzen werden durch folgende Methode bestimmt:

Als Maß für die Lipoxygenase-Hemmung wurde die Freisetzung von Leukotrien B₄(LTB₄) an polymorphkernigen Rattenleukozyten (PMN) nach Zugabe von Substanzen und Ca-Ionophor mittels reverse phase HPLC nach Borgeat, P. et al., Proc. Nat. Acad. Sci. 76, 2148 - 2152 (1979), bestimmt. Die in vivo-Aktivität der Verbindungen wurde mit dem Mäuseohr-Entzündungsmodell nach Young, J.M. et al., J. of Investigative Dermatology 82, 367 - 371, (1984) nachgewiesen.

Die neuen Wirkstoffe können in an sich bekannter Weise unter Verwendung inerter nichttoxischer, pharmazeutisch geeigneten Trägerstoffe oder Lösungsmittel in die üblichen Formulierungen überführt werden, wie Tabletten, Kapseln, Dragees, Pillen, Granulate, Aerosole, Sirupe, Emulsionen, Suspensionen und Lösungen. Hierbei soll die therapeutisch wirksame Verbindung jeweils in einer Konzentration von etwa 0,5 bis 90 Gew.-%, bevorzugt von 10 bis 70 Gew.-%, in der Zubereitung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nichttoxische organische Lösungsmittel wie Paraffine (z.B. Erdölfraktionen), pflanzliche Öle (z.B. Erdnuß/Sesamöl), Alkohole (z.B. Ethylalkohol, Glycerin), Glykole (z.B. Propylenglykol, Polyethylenglykol), feste Trägerstoffe, wie natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäureester, Polyoxyethylen-Fettalkohole-Ether, Alkylsulfonate und Arylsulfonate), Dispergiermittel (z.B. Lignin-Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation kann in üblicher Weise erfolgen, vorzugsweise oral oder parenteral, insbesondere perlingual oder intravenös. Im Falle der oralen Anwendung können Tabletten selbstverständlich außer den genannten Trägerstoffen auch Zusätze wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer den obengannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg, vorzugsweise etwa 0,01 bis 5 mg/kg Körpergewicht, zur Erzielung wirksamer Ergebnisse zu verabreichen. Bei oraler Applikation beträgt die Dosierung im allgemeinen etwa 0,1 bis 200 mg/kg, vorzugsweise 1 bis 100 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und zu dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Fall der Applikation größerer Mengen kann es empfehlenswert sein, diese in mehreren Einzelgaben über den Tag zu verteilen.

### Herstellungbeispiele

### Beispiel 1

### 1-[4-(Chinolin-2-yl-methoxy)phenyl]-1-pentylamin

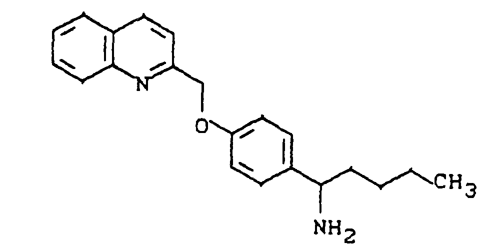

10,5 g (30 mmol) 2-[4-(Chinolin-2-yl-methoxy)phenyl]capronsäure und 7,7 ml (36 mmol) Phosphorsäurediphenylesterazid werden bei 0°C in 100 ml Dimethylformamid gelöst und 10 ml (72 mmol) Triethylamin zugegeben. Es wird 1 h auf 70°C erwärmt, auf 0°C gekühlt, 75 ml 5 normale Salzsäure zugetropft und wiederum 1 h auf 70°C erwärmt. Das Reaktionsgemisch wird auf Wasser gegeben und mit 2 normaler Natronlauge alkalisch gestellt und 5 mal mit Essigester extrahiert. Nach Trocknen über Na₂SO₄ wird eingedampft. Das Produkt kristallisiert aus.
Ausbeute: 8,9 g (92,6% der Theorie)
Festpunkt: 97-100°C

### Beispiel 2

### [4-(Chinolin-2-yl-methoxy)phenyl]-cyclopentyl-methylamin

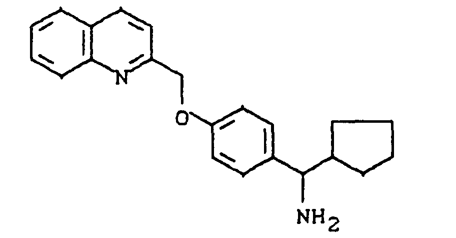

Die Darstellung erfolgt analog zu Beispiel 1 aus 5 g (13,8 mmol) 2-[4-(Chinolin-2-yl-methoxy)phenyl]-2-cyclopentylessigsäure und 4,57 g (16,6 mmol) Phosphorsäurediphenylesterazid.
Ausbeute: 3,29 g (71,7% der Theorie)
Fp.: 112°C

### Beispiel 3

### N-(4-Toluolsulfonaminocarbonyl)-1-[4-(chinolin-2-yl-methoxy)phenyl]-1-pentylamin

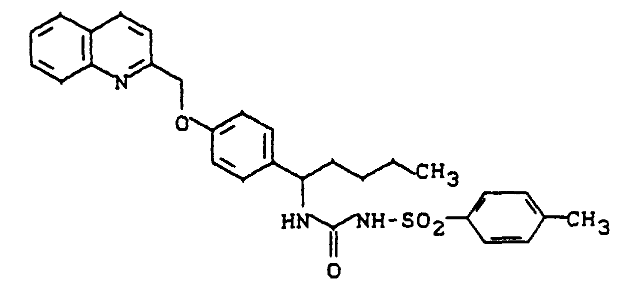

3,2 g (10 mmol) der Verbindung aus Beispiel 1 werden in 50 ml Methylenchlorid unter Schutzgas gelöst und mit 2,2 g (11 mmol) para-Toluolsulfonylisocyanat versetzt. Es wird 2 h bei 25°C gerührt, eingeengt und der Rückstand an Kieselgel 60 mit Methylenchlorid chromatographiert.
Ausbeute: 4,2 g (80% der Theorie)
Fp.: 141-144°C

### Beispiel 4

### N-(2-Toluolsulfonaminocarbonyl)-[4-(chinolin-2-yl-methoxy)phenyl]-cyclopentyl-methylamin

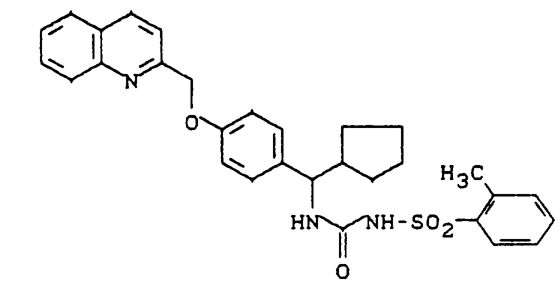

Die Darstellung erfolgt in Analogie zu der Vorschrift des Beispiels 3 aus 2,84 g (8,5 mmol) der Verbindung aus Beispiel 2 und 1,53 g (7,7 mmol) 2-Toluolsulfonylisocyanat.
Ausbeute: 3,4 g (83,5% der Theorie)
Fp.: 207-211°C

## Patentansprüche (Patentansprüche für folgende(n) Vertragsstaat(en): AT, BE, CH, DE, DK, FR, GB, GR, IT, LI, LU, NL, SE)

1. Substituierte N-(Chinolin-2-yl-methoxy)benzylsulfonylharnstoffe der allgemeinen Formel (I), 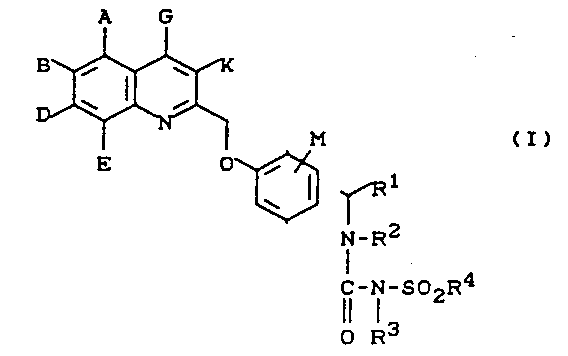 in welcher
A, B, D, E, G, K und M gleich oder verschieden sind und
- für Wasserstoff, Hydroxy, Halogen, Trifluormethyl, Trifluormethoxy oder Carboxy stehen,
- für geradkettiges oder verzweigtes Alkyl mit bis zu 10 Kohlenstoffatomen stehen, das gegebenenfalls durch Hydroxy oder Halogen substituiert ist,
- für geradkettiges oder verzweigtes Alkoxy oder Alkoxycarbonyl mit bis zu 10 Kohlenstoffatomen stehen, oder
- für Aryl mit 6 bis 10 Kohlenstoffatomen stehen, das gegebenenfalls durch Halogen, Nitro, Cyano oder durch geradkettiges oder verzweigtes Alkyl oder Alkoxy mit bis zu 8 Kohlenstoffatomen substituiert ist,
R¹
- für geradkettiges oder verzweigtes Alkyl mit bis zu 10 Kohlenstoffatomen steht, das gegebenenfalls durch Alkoxy mit bis zu 8 Kohlenstoffatomen, Halogen oder durch Cycloalkyl mit 3 bis 8 Kohlenstoffatomen substituiert ist,
- für Cycloalkyl mit 3 bis 8 Kohlenstoffatomen steht, das gegebenenfalls durch Halogen oder geradkettiges oder verzweigtes Alkyl mit bis zu 8 Kohlenstoffatomen substituiert ist,
R und R³ gleich oder verschieden sind und
- für Wasserstoff oder geradkettiges oder verzweigtes Alkyl mit bis zu 10 Kohlenstoffatomen stehen, das gegebenenfalls durch Halogen, Hydroxy oder durch Aryl mit 6 bis 10 Kohlenstoffatomen substituiert ist
R⁴
- für geradkettiges oder verzweigtes Alkyl mit bis zu 10 Kohlenstoffatomen steht, das gegebenenfalls durch Halogen, Nitro, Cyano, Hydroxy, Trifluormethyl oder durch Aryl mit 6 bis 10 Kohlenstoffatomen substituiert ist,
- für einen 5- bis 7-gliedrigen Heterocyclus mit bis zu 4 verschiedenen Heteroatomen aus der Reihe Schwefel, Sauerstoff oder Stickstoff, oder für Aryl mit 6 bis 10 Kohlenstoffatomen steht, wobei der Heterocyclus und der Arylrest gegebenenfalls bis zu 3-fach gleich oder verschieden durch Halogen, Nitro, Cyano, Hydroxy, geradkettiges oder verzweigtes Alkyl, Alkylthio oder Alkoxy mit bis zu 8 Kohlenstoffatomen, Trifluormethyl oder Trifluormethoxy substituiert sind,
und deren Salze.

2. Substituierte N-(Chinolin-2-yl-methoxy)benzylsulfonylharnstoffe der Formel nach Anspruch 1
worin
A, B, D, E, G, K und M gleich oder verschieden sind und
- für Wasserstoff, Fluor, Chlor, Trifluormethoxy oder Carboxy stehen,
- für geradkettiges oder verzweigtes Alkyl mit bis zu 8 Kohlenstoffatomen stehen, das gegebenenfalls durch Hydroxy, Fluor, Chlor oder Brom substituiert ist,
- für geradkettiges oder verzweigtes Alkoxy oder Alkoxycarbonyl mit bis zu 8 Kohlenstoffatomen stehen, oder
- - für Phenyl stehen, das gegebenenfalls durch Fluor, Chlor, Brom, Nitro, Cyano oder durch geradkettiges oder verzweigtes Alkyl oder Alkoxy mit bis zu 6 Kohlenstoffatomen substituiert ist,
R¹
- für geradkettiges oder verzweigtes Alkyl mit bis zu 8 Kohlenstoffatomen steht, das gegebenenfalls durch Hydroxy, Fluor, Chlor, Alkoxy mit bis zu 6 Kohlenstoffatomen oder durch Cyclopropyl, Cyclobutyl, Cyclopentyl, Cyclohexyl oder Cycloheptyl substituiert ist,
- für Cyclopropyl, Cyclobutyl, Cyclopentyl, Cyclohexyl oder Cycloheptyl steht, das gegebenenfalls durch Fluor, Chlor oder geradkettiges oder verzweigtes Alkyl mit bis zu 6 Kohlenstoffatomen substituiert ist,
R und R³ gleich oder verschieden sind und
- für Wasserstoff oder geradkettiges oder verzweigtes Alkyl mit bis zu 8 Kohlenstoffatomen stehen, das gegebenenfalls durch Fluor oder Chlor substituiert ist,
R⁴
- für geradkettiges oder verzweigtes Alkyl mit bis zu 8 Kohlenstoffatomen steht, das gegebenenfalls durch Fluor, Chlor, Nitro, Cyano, Hydroxy oder durch Phenyl substituiert ist,
- für Phenyl steht, das gegebenenfalls bis zu 2-fach gleich oder verschieden durch Fluor, Chlor, Brom, Nitro, Cyano, Hydroxy, geradkettiges oder verzweigtes Alkyl, Alkylthio oder Alkoxy mit bis zu 6 Kohlenstoffatomen, Trifluormethyl oder Trifluormethoxy substituiert ist
und deren Salze.

3. Substituierte N-(Chinolin-2-yl-methoxy)benzylsulfonylharnstoffe der Formel nach Anspruch 1
worin
A, B, D, E, G, K und M gleich oder verschieden sind und
- für Wasserstoff, Fluor, Chlor, geradkettiges oder verzweigtes Alkyl oder Alkoxy mit bis zu 6 Kohlenstoffatomen stehen,
R¹
- für geradkettiges oder verzweigtes Alkyl mit bis zu 6 Kohlenstoffatomen steht, das gegebenenfalls durch Fluor, Chlor oder Alkoxy mit bis zu 4 Kohlenstoffatomen oder durch Cyclopropyl, Cyclopentyl oder Cyclohexyl substituiert ist,
- für Cyclobutyl, Cyclopentyl, Cyclohexyl oder Cycloheptyl steht, das gegebenenfalls durch geradkettiges oder verzweigtes Alkyl mit bis zu 4 Kohlenstoffatomen oder durch Fluor substituiert ist,
R und R³ gleich oder verschieden sind und
- für Wasserstoff oder geradkettiges oder verzweigtes Alkyl mit bis zu 6 Kohlenstoffatomen stehen,
R⁴
- für geradkettiges oder verzweigtes Alkyl mit bis zu 6 Kohlenstoffatomen steht, das gegebenenfalls durch Fluor, Chlor oder Phenyl substituiert ist,
- für Phenyl steht, das gegebenenfalls durch Fluor, Chlor, Brom oder durch geradkettiges oder verzweigtes Alkyl mit bis zu 4 Kohlenstoffatomen substituiert ist
und deren Salze.

4. Substituierte N-(Chinolin-2-yl-methoxy)benzylsulfonylharnstoffe nach Anspruch 1 bis 3 zur Behandlung von Krankheiten.

5. Verfahren zur Herstellung von substituierten N-(Chinolin-2-yl-methoxy)benzyl-sulfonylharnstoffen nach Anspruch 1 bis 3, und deren Salze, dadurch gekennzeichnet, daß man
Amine der allgemeinen Formel 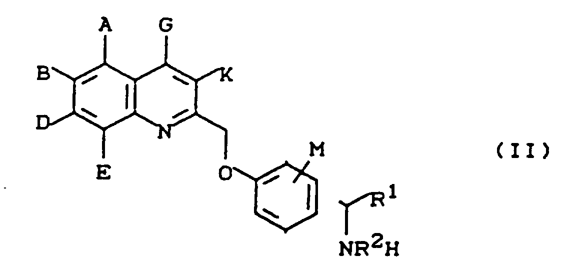 in welcher
A, B, D, E, G, K, M, R¹ und R die oben angegebene Bedeutung haben, mit Sulfonylisocyanaten der allgemeinen Formel
R⁴-SO₂-NCO (III)
in welcher
R⁴ die oben angegebene Bedeutung hat,
in einem inerten Lösemittel zu Verbindungen der allgemeinen Formel (Ia) 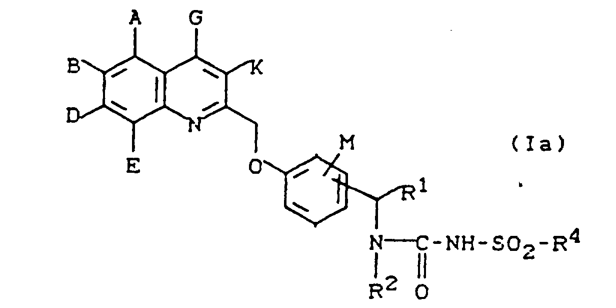 in welcher
A, B, D, E, G, K, M, R¹, R und R⁴ die oben angegebene Bedeutung haben,
umsetzt, und im Fall der Verbindungen der Formel (I) mit R³ ≠ H anschließend die Verbindungen der Formel (Ia) mit Alkylierungsmitteln in inerten Losemitteln alkyliert.

6. Arzneimittel enthaltend mindestens einen substituierten N-(Chinolin-2-yl-methoxy)benzyl-sulfonylharnstoff nach Anspruch 1.

7. Verfahren zur Herstellung eines Arnzeimittels nach Anspruch 6, dadurch gekennzeichnet, daß man substituierte N-(Chinolin-2-yl-methoxy)benzyl-sulfonylharnstoffe nach Anspruch 1, gegebenenfalls mit Hilfe geeigneter Hilfs- und Trägerstoffe in eine geeignete Applikationsform bringt.

8. Verwendung von substituierten N-(Chinolin-2-yl-methoxy)benzyl-sulfonylharnstoffen nach Anspruch 1 zur Herstellung von Arzneimitteln.

9. Verwendung von substituierten N-(Chinolin-2-yl-methoxy)benzyl-sulfonylharnstoffen nach Anspruch 1 zur Herstellung von Lipoxygenasehemmern.

## Patentansprüche (Patentansprüche für folgende(n) Vertragsstaat(en): ES)

1. Verfahren zur Herstellung von substituierten N-(Chinolin-2-yl-methoxy)benzyl-sulfonylharnstoffen der allgemeinen Formel 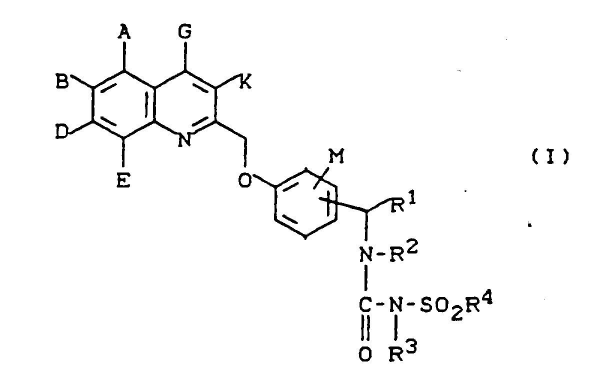 in welcher
A, B, D, E, G, K und M gleich oder verschieden sind und
- für Wasserstoff, Hydroxy, Halogen, Trifluormethyl, Trifluormethoxy oder Carboxy stehen,
- für geradkettiges oder verzweigtes Alkyl mit bis zu 10 Kohlenstoffatomen stehen, das gegebenenfalls durch Hydroxy oder Halogen substituiert ist,
- für geradkettiges oder verzweigtes Alkoxy oder Alkoxycarbonyl mit bis zu 10 Kohlenstoffatomen stehen, oder
- für Aryl mit 6 bis 10 Kohlenstoffatomen stehen, das gegebenenfalls durch Halogen, Nitro, Cyano oder durch geradkettiges.oder verzweigtes Alkyl oder Alkoxy mit bis zu 8 Kohlenstoffatomen substituiert ist,
R¹
- für geradkettiges oder verzweigtes Alkyl mit bis zu 10 Kohlenstoffatomen steht, das gegebenenfalls durch Alkoxy mit bis zu 8 Kohlenstoffatomen, Halogen oder durch Cycloalkyl mit 3 bis 8 Kohlenstoffatomen substituiert ist,
- für Cycloalkyl mit 3 bis 8 Kohlenstoffatomen steht, das gegebenenfalls durch Halogen oder geradkettiges oder verzweigtes Alkyl mit bis zu 8 Kohlenstoffatomen substituiert ist,
R und R³ gleich oder verschieden sind und
- für Wasserstoff oder geradkettiges oder verzweigtes Alkyl mit bis zu 10 Kohlenstoffatomen stehen, das gegebenenfalls durch Halogen, Hydroxy oder durch Aryl mit 6 bis 10 Kohlenstoffatomen substituiert ist,
R⁴
- für geradkettiges oder verzweigtes Alkyl mit bis zu 10 Kohlenstoffatomen steht, das gegebenenfalls durch Halogen, Nitro, Cyano, Hydroxy, Trifluormethyl oder durch Aryl mit 6 bis 10 Kohlenstoffatomen substituiert ist,
- für einen 5- bis 7-gliedrigen Heterocyclus mit bis zu 4 verschiedenen Heteroatomen aus der Reihe Schwefel, Sauerstoff oder Stickstoff, oder für Aryl mit 6 bis 10 Kohlenstoffatomen steht, wobei der Heterocyclus und der Arylrest gegebenenfalls bis zu 3-fach gleich oder verschieden durch Halogen, Nitro, Cyano, Hydroxy, geradkettiges oder verzweigtes Alkyl, Alkylthio oder Alkoxy mit bis zu 8 Kohlenstoffatomen, Trifluormethyl oder Trifluormethoxy substituiert sind,
und deren Salze, dadurch gekennzeichnet, daß man Amine der allgemeinen Formel 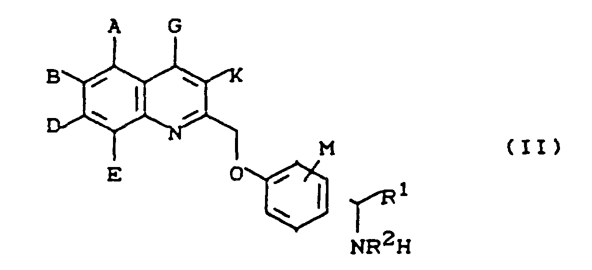 in welcher
A, B, D, E, G, K, M, R¹ und R die oben angegebene Bedeutung haben,
mit Sulfonylisocyanaten der allgemeinen Formel
R⁴-SO₂-NCO (III)
in welcher
R⁴ die oben angegebene Bedeutung hat,
in einem inerten Lösemittel zu Verbindungen der allgemeinen Formel (Ia) 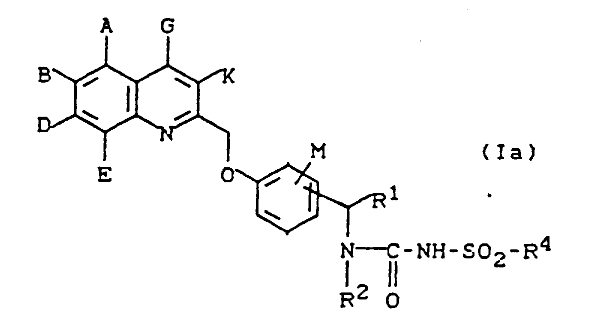 in welcher
A, B, D, E, G, K, M, R¹, R und R⁴ die oben angegebene Bedeutung haben,
umsetzt, und im Fall der Verbindungen der Formel (I) mit R³ ≠ H anschließend die Verbindungen der Formel (Ia) mit Alkylierungsmitteln in inerten Losemitteln alkyliert.

## Claims (Claims for the following Contracting State(s): AT, BE, CH, DE, DK, FR, GB, GR, IT, LI, LU, NL, SE)

1. Substituted N-(quinolin-2-yl-methoxy)benzyl-sulphonylureas of the general formula (I), 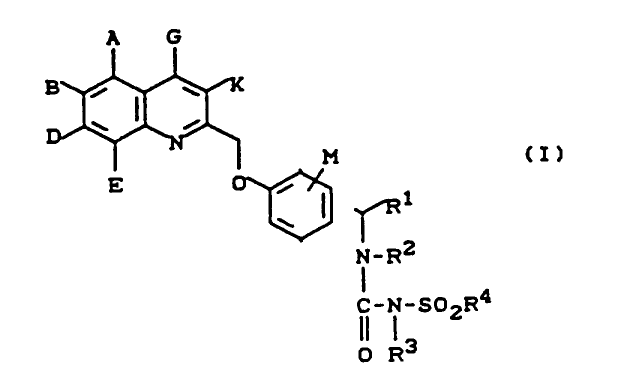 in which
A, B, D, E, G, K and Mare identical or different and
- represent hydrogen, hydroxyl, halogen, trifluoromethyl, trifluoromethoxy or carboxyl,
- represent straight-chain or branched alkyl having up to 10 carbon atoms, which is optionally substituted by hydroxyl or halogen,
- represent straight-chain or branched alkoxy or alkoxycarbonyl having up to 10 carbon atoms, or
- represent aryl having 6 to 10 carbon atoms, which is optionally substituted by halogen, nitro, cyano or by straight-chain or branched alkyl or alkoxy having up to 8 carbon atoms,
R¹
- represents straight-chain or branched alkyl having up to 10 carbon atoms, which is optionally substituted by alkoxy having up to 8 carbon atoms, halogen or cycloalkyl having 3 to 8 carbon atoms, or
- represents cycloalkyl having 3 to 8 carbon atoms, which is optionally substituted by halogen or straight-chain or branched alkyl having up to 8 carbon atoms,
R and R³ are identical or different and
- represent hydrogen or straight-chain or branched alkyl having up to 10 carbon atoms, which is optionally substituted by halogen, hydroxyl or by aryl having 6 to 10 carbon atoms, and
R⁴
- represents straight-chain or branched alkyl having up to 10 carbon atoms, which is optionally substituted by halogen, nitro, cyano, hydroxyl, trifluoromethyl or by aryl having 6 to 10 carbon atoms,
- or represents a 5- to 7-membered heterocyclic radical having up to 4 different heteroatoms from the series comprising sulphur, oxygen and nitrogen, or represents aryl having 6 to 10 carbon atoms, the heterocyclic radical and the aryl radical optionally being substituted by up to 3 identical or different substituents from the group comprising halogen, nitro, cyano, hydroxyl, straight-chain or branched alkyl, alkylthio and alkoxy having up to 8 carbon atoms, trifluoromethyl and trifluoromethoxy, and salts thereof.

2. Substituted N-(quinolin-2-yl-methoxy)benzyl-sulphonylureas of the formula according to Claim 1,
wherein
A, B, D, E, G, K and Mare identical or different and
- represent hydrogen, fluorine, chlorine, trifluoromethoxy or carboxyl,
- represent straight-chain or branched alkyl having up to 8 carbon atoms, which is optionally substituted by hydroxyl, fluorine, chlorine or bromine,
- represent straight-chain or branched alkoxy or alkoxycarbonyl having up to 8 carbon atoms, or
- represent phenyl, which is optionally substituted by fluorine, chlorine, bromine, nitro, cyano or by straight-chain or branched alkyl or alkoxy having up to 6 carbon atoms,
R¹
- represents straight-chain or branched alkyl having up to 8 carbon atoms, which is optionally substituted by hydroxyl, fluorine, chlorine, alkoxy having up to 6 carbon atoms or by cyclopropyl, cyclobutyl, cyclopentyl, cyclohexyl or cycloheptyl,
- or represents cyclopropyl, cyclobutyl, cyclopentyl, cyclohexyl or cycloheptyl, which is optionally substituted by fluorine, chlorine, or straight-chain or branched alkyl having up to 6 carbon atoms,
R and R³ are identical or different and
- represent hydrogen or straight-chain or branched alkyl having up to 8 carbon atoms, which is optionally substituted by fluorine or chlorine, and
R⁴
- represents straight-chain or branched alkyl having up to 8 carbon atoms, which is optionally substituted by fluorine, chlorine, nitro, cyano, hydroxyl or by phenyl,
- or represents phenyl, which is optionally substituted by up to 2 identical or different substituents from the group comprising fluorine, chlorine, bromine, nitro, cyano, hydroxyl, straight-chain or branched alkyl, alkylthio and alkoxy having up to 6 carbon atoms, trifluoromethyl and trifluoromethoxy,
and salts thereof.

3. Substituted N-(quinolin-2-yl-methoxy)benzyl-sulphonylureas of the formula according to Claim 1,
wherein
A, B, D, E, G, K and M are identical or different and
- represent hydrogen, fluorine, chlorine, or straight-chain or branched alkyl or alkoxy having up to 6 carbon atoms,
R¹
- represents straight-chain or branched alkyl having up to 6 carbon atoms, which is optionally substituted by fluorine, chlorine or alkoxy having up to 4 carbon atoms or by cyclopropyl, cyclopentyl or cyclohexyl,
- or represents cyclobutyl, cyclopentyl, cyclohexyl or cycloheptyl, which is optionally substituted by straight-chain or branched alkyl having up to 4 carbon atoms or by fluorine,
R and R³ are identical or different and
- represent hydrogen or straight-chain or branched alkyl having up to 6 carbon atoms, and
R⁴
- represents straight-chain or branched alkyl having up to 6 carbon atoms, which is optionally substituted by fluorine, chlorine or phenyl,
- or represents phenyl, which is optionally substituted by fluorine, chlorine, bromine or by straight-chain or branched alkyl having up to 4 carbon atoms,
and salts thereof.

4. Substituted N-(quinolin-2-yl-methoxy)benzyl-sulphonylureas according to Claims 1 to 3 for the treatment of diseases.

5. Process for the preparation of substituted N-(quinolin-2-yl-methoxy)benzyl-sulphonylureas according to Claim 1 to 3,
and salts thereof, characterized in that
amines of the general formula 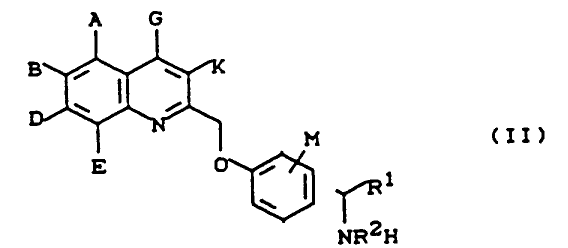 in which
A, B, D, E, G, K, M, R¹ and R have the abovementioned meaning,
are reacted with sulphonyl isocyanates of the general formula (III)
R⁴-SO₂-NCO (III)
in which
R⁴ has the abovementioned meaning,
in an inert solvent to give compounds of the general formula (Ia) 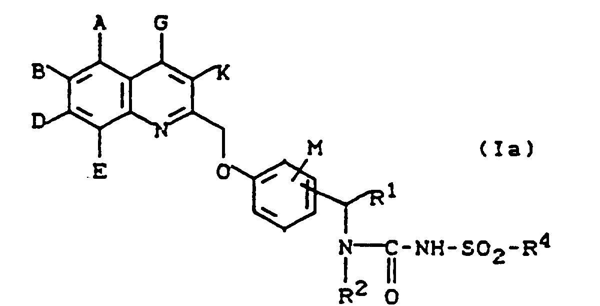 in which
A, B, D, E, G, K, M, R¹, R and R⁴ have the abovementioned meaning,
and in the case of the compounds of the formula (I) where R³ ≠ H, the compounds of the formula (Ia) are then alkylated with alkylating agents in inert solvents.

6. Medicaments containing at least one substituted N-(quinolin-2-yl-methoxy)benzyl-sulphonylurea according to Claim 1.

7. Process for the preparation of a medicament according to Claim 6, characterized in that substituted N-(quinolin-2-yl-methoxy)benzyl-sulphonylureas according to Claim 1 are brought into a suitable administration form, if appropriate with the aid of suitable auxiliaries and excipients.

8. Use of substituted N-(quinolin-2-yl-methoxy)benzyl-sulphonylureas according to Claim 1 for the preparation of medicaments.

9. Use of substituted N- (quinolin-2-yl-methoxy)benzyl-sulphonylureas according to Claim 1 for the preparation of lipoxygenase inhibitors.

## Claims (Claims for the following Contracting State(s): ES)

1. Process for the preparation of substituted N-(quinolin-2-yl-methoxy)benzyl-sulphonylureas of the general formula 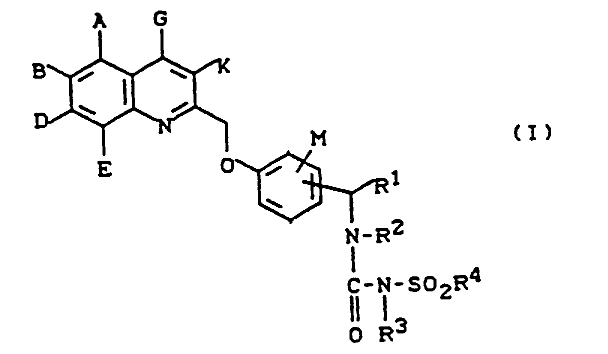 in which
A, B, D, E, G, K and M are identical or different and
- represent hydrogen, hydroxyl, halogen, trifluoromethyl, trifluoromethoxy or carboxyl,
- represent straight-chain or branched alkyl having up to 10 carbon atoms, which is optionally substituted by hydroxyl or halogen,
- represent straight-chain or branched alkoxy or alkoxycarbonyl having up to 10 carbon atoms, or
- represent aryl having 6 to 10 carbon atoms, which is optionally substituted by halogen, nitro, cyano or by straight-chain or branched alkyl or alkoxy having up to 8 carbon atoms,
R¹
- represents straight-chain or branched alkyl having up to 10 carbon atoms, which is optionally substituted by alkoxy having up to 8 carbon atoms, halogen or cycloalkyl having 3 to 8 carbon atoms, or
- represents cycloalkyl having 3 to 8 carbon atoms, which is optionally substituted by halogen or straight-chain or branched alkyl having up to 8 carbon atoms,
R and R³ are identical or different and
- represent hydrogen or straight-chain or branched alkyl having up to 10 carbon atoms, which is optionally substituted by halogen, hydroxyl or by aryl having 6 to 10 carbon atoms, and
R⁴
- represents straight-chain or branched alkyl having up to 10 carbon atoms, which is optionally substituted by halogen, nitro, cyano, hydroxyl, trifluoromethyl or by aryl having 6 to 10 carbon atoms,
- or represents a 5- to 7-membered heterocyclic radical having up to 4 different heteroatoms from the series comprising sulphur, oxygen and nitrogen, or represents aryl having 6 to 10 carbon atoms, the heterocyclic radical and the aryl radical optionally being substituted by up to 3 identical or different substituents from the group comprising halogen, nitro, cyano, hydroxyl, straight-chain or branched alkyl, alkylthio and alkoxy having up to 8 carbon atoms, trifluoromethyl and trifluoromethoxy, and salts thereof, characterized in that amines of the general formula 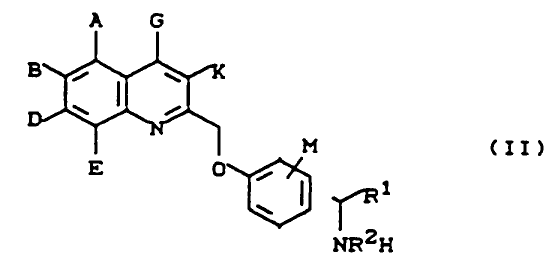 in which
A, B, D, E, G, K, M, R¹ and R have the abovementioned meaning,
are reacted with sulphonyl isocyanates of the general formula (III)
R⁴-SO₂-NCO (III)
in which
R⁴ has the abovementioned meaning,
in an inert solvent to give compounds of the general formula (Ia) 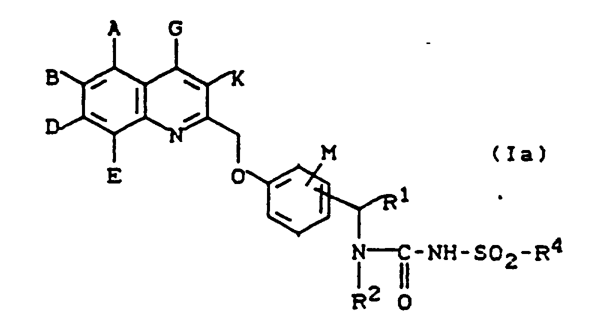 in which
A, B, D, E, G, K, M, R¹, R and R⁴ have the abovementioned meaning,
and in the case of the compounds of the formula (I) where R³ ≠ H, the compounds of the formula (Ia) are then alkylated with alkylating agents in inert solvents.

## Revendications (Revendications pour l'(les) Etat(s) contractant(s) suivant(s): AT, BE, CH, DE, DK, FR, GB, GR, IT, LI, LU, NL, SE)

1. N-(quinoléine-2-yl-méthoxy)benzylsulfonylurées substituées de formule générale (I) 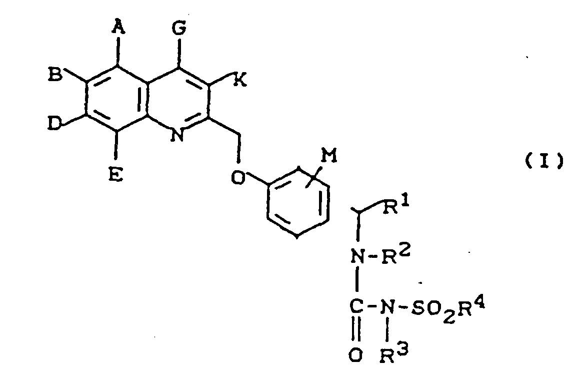 dans laquelle
A, B, D, E, G, K et M sont identiques ou différents et représentent
- de l'hydrogène, un groupe hydroxy, un halogène, un groupe trifluorométhyle, trifluorométhoxy ou carboxy,
- un groupe alkyle linéaire ou ramifié ayant jusqu'à 10 atomes de carbone, qui est substitué le cas échéant par un radical hydroxy ou un halogène,
- un groupe alkoxy ou alkoxycarbonyle linéaire ou ramifié ayant jusqu'à 10 atomes de carbone, ou
- un groupe aryle ayant 6 à 10 atomes de carbone, qui est substitué le cas échéant par un halogène, un radical nitro, cyano ou par un radical alkyle ou alkoxy linéaire ou ramifié ayant jusqu'à 8 atomes de carbone,
R¹ représente
- un groupe alkyle linéaire ou ramifié ayant jusqu'à 10 atomes de carbone, qui est substitué le cas échéant par un radical alkoxy ayant jusqu'à 8 atomes de carbone, un halogène ou un radical cycloalkyle ayant 3 à 8 atomes de carbone,
- un groupe cycloalkyle ayant 3 à 8 atomes de carbone, qui est substitué le cas échéant par un halogène ou par un radical alkyle linéaire ou ramifié ayant jusqu'à 8 atomes de carbone,
R et R³ sont identiques ou différents et représentent
- de l'hydrogène ou un groupe alkyle linéaire ou ramifié ayant jusqu'à 10 atomes de carbone, qui est substitué le cas échéant par un halogène, un radical hydroxy ou par un radical aryle ayant 6 à 10 atomes de carbone,
R⁴ représente
- un groupe alkyle linéaire ou ramifié ayant jusqu'à 10 atomes de carbone, qui est substitué le cas échéant par un halogène, un radical nitro, cyano, hydroxy, trifluorométhyle ou par un radical aryle ayant 6 à 10 atomes de carbone,
- un hétérocycle pentagonal à heptagonal ayant jusqu'à 4 hétéroatomes différents de la série soufre, oxygène ou azote, ou un groupe aryle ayant 6 à 10 atomes de carbone, l'hétérocycle et le reste aryle portant éventuellement jusqu'à 3 substituants, identiques ou différents, halogéno, nitro, cyano, hydroxy, alkyle, alkylthio ou alkoxy linéaire ou ramifié ayant jusqu'à 8 atomes de carbone, trifluorométhyle ou trifluorométhoxy,
et leurs sels.

2. N- (quinoléine-2-yl-méthoxy)benzylsulfonylurées substituées de formule suivant la revendication 1, dans laquelle
A, B, D, E, G, K et M sont identiques ou différents et représentent :
- de l'hydrogène, du fluor, du chlore, un groupe trifluorométhoxy ou carboxy,
- un groupe alkyle linéaire ou ramifié ayant jusqu'à 8 atomes de carbone, qui est éventuellement substitué par un radical hydroxy, du fluor, du chlore ou du brome,
- un groupe alkoxy ou alkoxycarbonyle linéaire ou ramifié ayant jusqu'à 8 atomes de carbone, ou bien
- un groupe phényle qui est substitué le cas échéant par du fluor, du chlore, du brome, un radical nitro, cyano ou par un radical alkyle ou alkoxy linéaire ou ramifié ayant jusqu'à 6 atomes de carbone,
R¹ représente
- un groupe alkyle linéaire ou ramifié ayant jusqu'à 8 atomes de carbone, qui est substitué le cas échéant par un radical hydroxy, fluoro, chloro, alkoxy ayant jusqu'à 6 atomes de carbone ou par un radical cyclopropyle, cyclobutyle, cyclopentyle, cyclohexyle ou cycloheptyle,
- un groupe cyclopropyle, cyclobutyle, cyclopentyle, cyclohexyle ou cycloheptyle qui est substitué le cas échéant par du fluor, du chlore ou un radical alkyle linéaire ou ramifié ayant jusqu'à 6 atomes de carbone,
R et R³ sont identiques ou différents et représentent
- de l'hydrogène ou un groupe alkyle linéaire ou ramifié ayant jusqu'à 8 atomes de carbone, qui est éventuellement substitué par du fluor ou du chlore,
R⁴ représente
- un groupe alkyle linéaire ou ramifié ayant jusqu'à 8 atomes de carbone, qui est substitué le cas échéant par du fluor, du chlore, un radical nitro, cyano, hydroxy ou par un radical phényle,
- un groupe phényle qui porte éventuellement jusqu'à 2 substituants, identiques ou différents, fluoro, chloro, bromo, nitro, cyano, hydroxy, alkyle, alkylthio ou alkoxy linéaire ou ramifié ayant jusqu'à 6 atomes de carbone, trifluorométhyle ou trifluorométhoxy,
et leurs sels.

3. N-(quinoléine-2-yl-méthoxy)benzylsulfonylurées substituées de formule suivant la revendication 1, dans laquelle
A, B, D, E, G, K et M sont identiques ou différents et représentent
- de l'hydrogène, du fluor, du chlore, un radical alkyle ou alkoxy linéaire ou ramifié ayant jusqu'à 6 atomes de carbone,
R¹ représente
- un groupe alkyle linéaire ou ramifié ayant jusqu'à 6 atomes de carbone, qui est substitué le cas échéant par du fluor, du chlore ou un radical alkoxy ayant jusqu'à 4 atomes de carbone ou par un radical cyclopropyle, cyclopentyle ou cyclohexyle,
- un groupe cyclobutyle, cyclopentyle, cyclohexyle ou cycloheptyle qui est substitué le cas échéant par un radical alkyle linéaire ou ramifié ayant jusqu'à 4 atomes de carbone ou par du fluor,
R et R³ sont identiques ou différents et représentent
- de l'hydrogène ou un radical alkyle linéaire ou ramifié ayant jusqu'à 6 atomes de carbone,
R⁴
- est un radical alkyle linéaire ou ramifié ayant jusqu'à 6 atomes de carbone, qui est substitué le cas échéant par du fluor, du chlore ou un radical phényle,
- un groupe phényle qui est substitué le cas échéant par du fluor, du chlore, du brome ou un radical alkyle linéaire ou ramifié ayant jusqu'à 4 atomes de carbone,
et leurs sels.

4. N-(quinoléine-2-yl-méthoxy)benzylsulfonylurées substituées suivant les revendications 1 à 3, destinées au traitement de maladies.

5. Procédé de production de N-(quinoléine-2-yl-méthoxy)benzylsulfonylurées substituées suivant les revendications 1 à 3, et de leurs sels, caractérisé en ce que :
on fait réagir des amines de formule générale 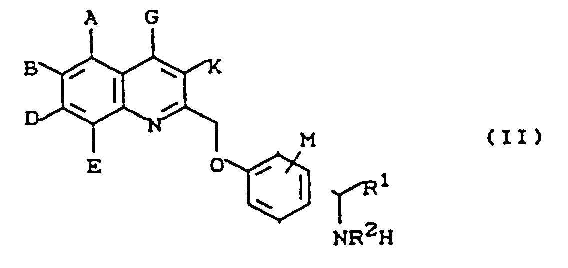 dans laquelle
A, B, D, E, G, K, M, R et R ont la définition indiquée ci-desssus,
avec des sulfonylisocyanates de formule générale
R⁴-SO₂-NCO (III)
dans laquelle
R⁴ a la définition indiquée ci-dessus,
dans un solvant inerte pour former des composés de formule générale (Ia) 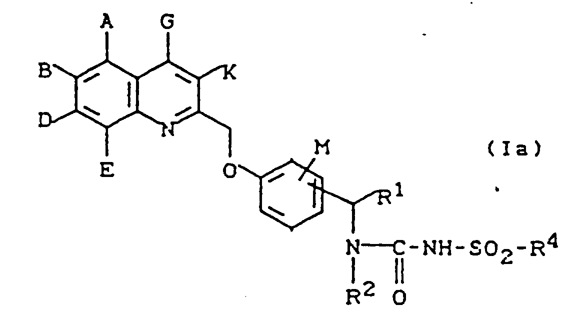 dans laquelle
A, B, D, E, G, K, M, R¹, R et R⁴ ont la définition indiquée ci-dessus,
et dans le cas de composés de fromule (I) dans laquelle R³ est différent d'un atome d'hydrogène, on alkyle ensuite les composés de formule (Ia) avec des agents alkylants dans des solvants inertes.

6. Médicament contenant au moins une N-(quinoléine-2-yl-méthoxy)benzylsulfonylurée substituée suivant la revendication 1.

7. Procédé de préparation d'un médicament suivant la revendication 6, caractérisé en ce qu'on fait prendre une forme administrable appropriée à des N-(quinoléine-2-yl-méthoxy)benzylsulfonylurées substituées suivant la revendication 1, le cas échéant à l'aide de substances auxiliaires et de supports appropriés.

8. Utilisation de N-(quinoléine-2-yl-méthoxy)-benzylsulfonylurées substituées suivant la revendication 1 pour la préparation de médicaments.

9. Utilisation de N-(quinoléine-2-yl-méthoxy)-benzylsulfonylurées substituées suivant la revendication 1 pour la préparation d'inhibiteurs de lipoxygénase.

## Revendications (Revendications pour l'(les) Etat(s) contractant(s) suivant(s): ES)

1. Procédé de production de N-(quinoléine-2-yl-méthoxy)benzylsulfonylurées substituées de formule générale 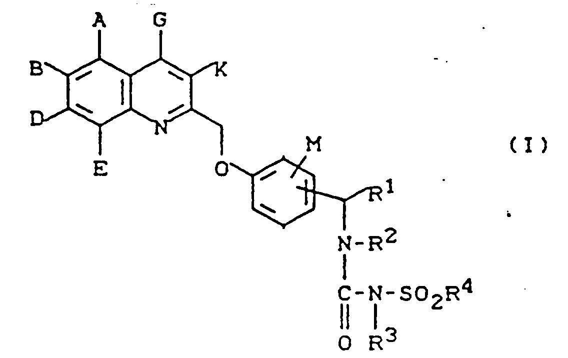 dans laquelle
A, B, D, E, G, K et M sont identiques ou différents et représentent
- de l'hydrogène, un groupe hydroxy, un halogène, un groupe trifluorométhyle, trifluorométhoxy ou carboxy,
- un groupe alkyle linéaire ou ramifié ayant jusqu'à 10 atomes de carbone, qui est substitué le cas échéant par un radical hydroxy ou un halogène,
- un groupe alkoxy ou alkoxycarbonyle linéaire ou ramifié ayant jusqu'à 10 atomes de carbone, ou
- un groupe aryle ayant 6 à 10 atomes de carbone, qui est substitué le cas échéant par un halogène, un radical nitro, cyano ou par un radical alkyle ou alkoxy linéaire ou ramifié ayant jusqu'à 8 atomes de carbone,
R¹ représente
- un groupe alkyle linéaire ou ramifié ayant jusqu'à 10 atomes de carbone, qui est substitué le cas échéant par un radical alkoxy ayant jusqu'à 8 atomes de carbone, un halogène ou un radical cycloalkyle ayant 3 à 8 atomes de carbone,
- un groupe cycloalkyle ayant 3 à 8 atomes de carbone, qui est substitué le cas échéant par un halogène ou par un radical alkyle linéaire ou ramifié ayant jusqu'à 8 atomes de carbone,
R et R³ sont identiques ou différents et représentent
- de l'hydrogène ou un groupe alkyle linéaire ou ramifié ayant jusqu'à 10 atomes de carbone, qui est substitué le cas échéant par un halogène, un radical hydroxy ou par un radical aryle ayant 6 à 10 atomes de carbone,
R⁴ représente
- un groupe alkyle linéaire ou ramifié ayant jusqu'à 10 atomes de carbone, qui est substitué le cas échéant par un halogène, un radical nitro, cyano, hydroxy, trifluorométhyle ou par un radical aryle ayant 6 à 10 atomes de carbone,
- un hétérocycle pentagonal à heptagonal ayant jusqu'à 4 hétéroatomes différents de la série soufre, oxygène ou azote, ou un groupe aryle ayant 6 à 10 atomes de carbone, l'hétérocycle et le reste aryle portant éventuellement jusqu'à 3 substituants, identiques ou différents, halogéno, nitro, cyano, hydroxy, alkyle, alkylthio ou alkoxy linéaire ou ramifié ayant jusqu'à 8 atomes de carbone, trifluorométhyle ou trifluorométhoxy,
et de leurs sels, caractérisé en ce que :
on fait réagir des amines de formule générale 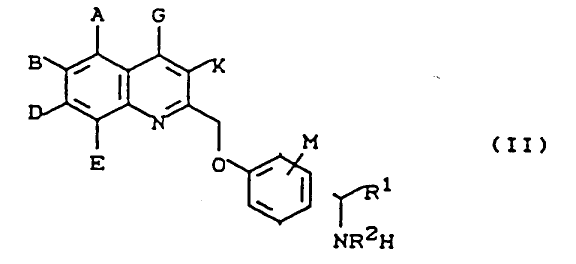 dans laquelle
A, B, D, E, G, K, M, R et R ont la définition indiquée ci-desssus,
avec des sulfonylisocyanates de formule générale
R⁴-SO₂-NCO (III)
dans laquelle
R⁴ a la définition indiquée ci-dessus,
dans un solvant inerte pour former des composés de formule générale (Ia) 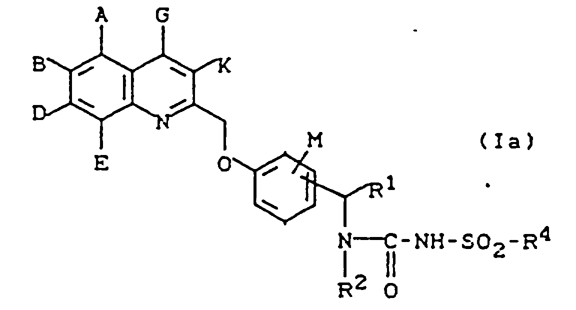 dans laquelle
A, B, D, E, G, K, M, R¹, R et R⁴ ont la définition indiquée ci-dessus,
et dans le cas de composés de fromule (I) dans laquelle R³ est différent d'un atome d'hydrogène, on alkyle ensuite les composés de formule (Ia) avec des agents alkylants dans des solvants inertes.
